(19) Europäisches Patentamt
European Patent Office
Office européen des brevets

(11) **EP 2 200 498 B1**

(12) **EUROPEAN PATENT SPECIFICATION**

(45) Date of publication and mention
of the grant of the patent:
**27.03.2013 Bulletin 2013/13**

(51) Int Cl.:
*A61B 3/14* *(2006.01)*     *A61B 3/12* *(2006.01)*
*A61B 3/18* *(2006.01)*

(21) Application number: **08838600.8**

(22) Date of filing: **16.10.2008**

(86) International application number:
**PCT/FI2008/050581**

(87) International publication number:
**WO 2009/050339 (23.04.2009 Gazette 2009/17)**

(54) **ILLUMINATING AN ORGAN**

BELEUCHTUNG EINES ORGANS

ECLAIRAGE D'UN ORGANE

(84) Designated Contracting States:
**AT BE BG CH CY CZ DE DK EE ES FI FR GB GR
HR HU IE IS IT LI LT LU LV MC MT NL NO PL PT
RO SE SI SK TR**

(30) Priority: **19.10.2007 FI 20075738**

(43) Date of publication of application:
**30.06.2010 Bulletin 2010/26**

(73) Proprietor: **Optomed Oy**
**90100 Oulu (FI)**

(72) Inventor: **POHJANEN, Petri**
**FI-90420 Oulu (FI)**

(74) Representative: **Karppinen, Olavi Arto**
**Kolster Oy Ab**
**P.O. Box 148**
**(Iso Roobertinkatu 23)**
**00121 Helsinki (FI)**

(56) References cited:
| | |
|---|---|
| **WO-A1-2004/000109** | **WO-A1-2005/044098** |
| **WO-A1-2007/076479** | **US-A- 4 932 774** |
| **US-A- 5 155 509** | **US-A1- 2002 003 608** |
| **US-A1- 2005 041 207** | **US-A1- 2005 200 707** |
| **US-A1- 2005 225 722** | **US-A1- 2006 177 205** |

## Description

### FIELD

The invention relates to a method and an examination device with which an organ under examination is illuminated.

### BACKGROUND

While examining different organs, such as the eye, ear, nose, mouth, etc., a digital examination device may be used, which forms an electric image that can be transferred to be displayed on a computer screen, for example. There may be a separate examination device for each organ, but the examination device may also comprise a common digital camera unit for examining different organs, and a plurality of optical components which can be attached to and detached from the camera unit and which act as objectives for the camera unit. The different optical components are in this case intended for forming an image of different organs, which makes the examination effective. Patent document US 20020003608 presents an ophthalmic examination apparatus comprising a plurality of portable ophtalmic diagnostic equipment devices having different ophtalmic functions. In particular, a slit lamp mechanically coupled to a support base is disclosed, comprising an optical radiation source arranged to direct optical radiation at an optical radiation structure located non axially to the optical axis. Patent document US 5155509 presents an oblique illumination device for use with an ophthalmic microscope. Patent document US 4932774 presents an illumination system of ophtalmological instrument. Patent document WO 2007076479 presents a pupil reflection eye tracking system and method.

However, the use of optical components attachable to and detachable from the camera unit is associated with problems. Although image-forming optics can be arranged according to the object under examination, illuminating of the object under examination is inadequate, since different objects under examination are illuminated by the same sources in the same way. Usually the illumination in digital systems must be implemented according to the exposure capability of a digital cell acting as a detector. As a consequence, optical radiation not adapted to the object and directed at the object under examination seldom brings out desired properties of the object properly, nor does it illuminate the region around the object sufficiently. Thus, the illumination of the object under examination is not optimised and may in some cases be quite insufficient for the intensity and band of the optical radiation.

### BRIEF DESCRIPTION

It is an object of the invention to provide an improved method and a device implementing the method.

This is achieved by a method for illuminating an organ, wherein a camera unit is used for forming an electric image of the organ. The method may employ at least one optical component, which is connectable to the camera unit and comprises at least one optical radiation source and at least one optical radiation control structure, each optical component including a data structure containing information associated with the optical component; directing optical radiation with the at least one optical radiation source to the at least one optical radiation control structure, which is located non-axially to the optical axis of the optical component; directing optical radiation with each optical radiation control structure at the organ under examination in a direction diverging from the optical axis of the optical component transferring information associated with the optical component from the data structure to the camera unit when at least one optical compo-nent is connected to the camera unit; and controlling the formation of an image of the organ by the camera unit on the basis of the information associated with one or more optical components.

The invention also relates to a device for forming an image of an organ, the device comprising a camera unit for forming an electric image of the organ. The device comprises a group of optical components, the group comprising at least one optical component, each optical component being connectable to the camera unit; each optical component comprises at least one optical radiation source, at least one optical radiation control structure and a data structure containing information associated with the optical component; the device is arranged to transfer information associated with the optical component from the data structure to the camera unit when at least one optical component is con-nected to the camera unit; the at least one optical radiation source is arranged to direct optical radiation at the at least one optical radiation control structure located non-axially to the optical axis of the optical component; and each optical radiation control structure is arranged to direct optical radiation from the optical radiation source at the organ in a direction diverging from the optical axis of the optical component; and the device is arranged to control the formation of an image of the organ by the camera unit on the basis of the information associated with one or more optical components.

Preferred embodiments of the invention are disclosed in the dependent claims.

The method and system of the invention provide a plurality of advantages. Radiation from an optical radiation source in an optical component is emitted to the object under examination in a direction diverging from the optical axis of the optical component in order to form a good image of the object under examination. Since each optical component is intended for examining and illuminating a specific organ, the object under examination can be illuminated as desired.

LIST OF FIGURES

The invention is now described in closer detail in connection with the preferred embodiments and with reference to the accompanying drawings, in which

Figure 1 shows an examination device,
Figure 2 shows a camera unit, to which an optical component is attached,
Figure 3 shows an optical component with an optical radiation source,
Figure 4 shows an optical component with two optical radiation sources,
Figure 5A shows an optical component with an optical radiation source and two optical radiation control structures,
Figure 5B shows a digital signal processor,
Figure 6 shows optical radiation feedback,
Figure 7 shows a camera unit, to which two optical components are connected,
Figure 8 shows a block diagram of the examination device,
Figure 9 shows the camera unit in a docking station, and
Figure 10 shows a flow diagram of the method.

DESCRIPTION OF EMBODIMENTS

According to the object under examination, the examination device may be connected with one or more optical components with suitable imaging optics. The optical components may communicate with each other and the rest of the equipment, and by utilizing the communication, optical radiation sources in both the lenses themselves and the frame of the device may be used in a controlled manner in all objects of which an image is formed in such a manner that radiation from all or some of the available optical radiation sources can be directed at the object under examination, controlled in a desired manner according to the object of which an image is formed. In this application, optical radiation refers to a wavelength band of approximately 100 nm to 500 $\mu$m.

For the most parts, a camera unit of the examination device may be similar to the solutions disclosed in Finnish Patents FI 107120, FI 200212233 and FI 20075499, wherefore the present application does not disclose features known per se of the camera unit in greater detail but expressly concentrates on the features of disclosed solution that differ from both the above facts and the prior art.

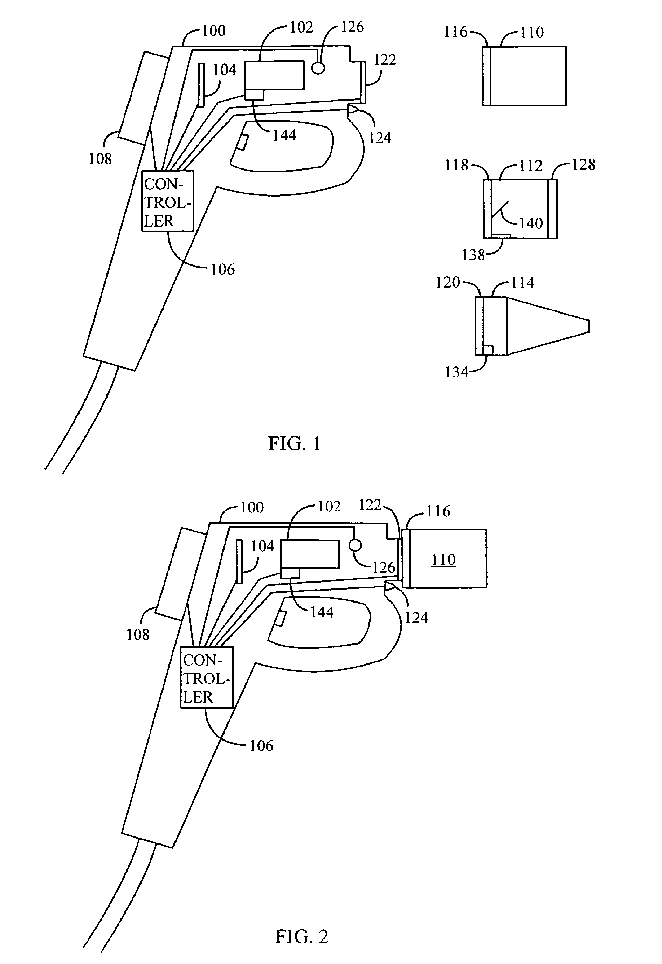

Let us first view the examination device generally by means of Figure 1. In this example the examination device is a camera unit 100, which may be a portable digital camera. The camera unit 100 of the examination device may comprise an optics unit 102, which may participate in forming an image of an organ to a detector 104 of the camera unit 100. The forming of an image to the detector 104 by means of the optics unit 102 can be adjusted by a motor 144, which may be controlled by a controller 106. When the examination device is in operation, the detector 104 may form an electric image of the organ. The image formed by the detector 104 may be supplied to the camera unit's 100 controller 106, which may comprise a processor and memory, for controlling the camera unit 100 and processing and storing the image and feasible other information. From the controller 106, the image may be supplied to a display 108 of the camera unit 100 for displaying the image and feasible other data. The detector 104 of the camera unit 100 may be a CCD (Charge Coupled Device) or CMOS cell (Complementary Metal Oxide Semiconductor), and the camera unit 100 may form still pictures or video image.

In addition to the camera unit 100, the examination device comprises at least one optical component 110 to 114, which is connectable to the camera unit 100. Each optical component 110 to 114 is intended, either alone or together with at least one other optical component 110 to 114, for forming an image of a predetermined organ. The at least one optical component 110 to 114 comprises at least one lens or mirror, which may, together with the optics unit 102, form an image of the organ, such as the eye, to the detector 104. An optical component suitable for the object under examination may be attached or added to or replaced in the camera unit 100. Attached to the camera unit 100, each of these optical components 110 to 114 may communicate with the camera unit 100 and/or with one another by using a data structure 116 to 120. Furthermore, it is possible that each optical component 110 to 114 communicates with devices in the surroundings. Each optical component 110 to 114, alone or together with one or more other optical components 110 to 114, may control the production, processing and storing of the image.

The data structure 116 to 120 of each optical component 110 to 114 may contain information on the optical component 110 to 114. The data structure 110 to 114 may be located in the frame of the optical component 110 to 114 or in at least one component used for forming an image, such as a lens. The optical component 110 to 114 may comprise, for instance, one or more elements forming an image, such as a lens or a mirror, and the optical component 110 to 114 may act as an additional objective of the camera unit 100.

The data structure 116 to 120 may be for instance an electromechanical structure, which attaches the optical component 110 to 114 mechanically to the camera unit 100 and establishes an electric connection between the camera unit 100 and the optical component 110 to 114. By connecting the data structure 116 to 120 against a counterpart 122 in the camera unit 100, the information associated with the optical component 110 to 114 may be transferred from the data structure 116 to 120 via the counterpart 122 to the controller 106 along a conductor, for instance. In this case, the data structure 116 to 120 and the counterpart 122 of the camera unit 100 may comprise one or more electric contact surfaces.

The electrical connection may be specific to each optical component 110 to 114 or component type. Through the contact surfaces, the camera unit 100 may switch on the electricity in the data structure 116 to 120, and the response of the data structure 116 to 120 to the electric signal of the camera unit 100 contains information characteristic of each optical component 110 to 114.

There may be a different optical component 110 to 114 for forming an image of different organs, in which case each optical component 110 to 114 has a different kind of connection. The connections may differ from one another in terms of, for instance, resistance, capacitance or inductance, which affects for example the current or voltage detected by the camera unit 100. Instead of such analogue coding, digital coding may also be used for separating the optical components 110 to 114 from one another.

The data structure 116 to 120 may also be, for example, a memory circuit comprising information characteristic of each optical component 110 to 114. The data structure 116 to 120 may be, for instance, a USB memory and, as the counterpart 122, the camera unit 100 may have a connector for the USB memory. The information associated with the optical component 110 to 114 may be transferred from the memory circuit to the controller 106 of the camera unit 100, which may use this information to control the camera unit 100 and an optical radiation source 300, 304 of each optical component 110 to 114 and thus the optical radiation.

Reading of the information included in the data structure 116 to 120 does not necessarily require a galvanic contact between the camera unit 100 and the data structure 116 to 120. The information associated with the optical component 110 to 114 may in this case be read from the data structure 116 to 120 capacitively, inductively or optically, for instance. The data structure 116 to 120 may be a bar code, which is read by a bar code reader of the camera unit 100. The bar code may also be read from the formed image by means of an image processing program of the camera unit 100. The bar code may be detected at a wavelength different from the wavelength at which an image is formed of the organ. The bar code may be identified by means of infrared radiation, for example, when an image of the organ is formed with visible light. Thus, the bar code does not interfere with the forming of an image of the organ.

The data structure 116 to 120 may also be an optically detectable property of each optical component 110 to 114, such as an image aberration, which may be e.g. a spherical aberration, astigmatism, coma, curvature of image field, distortion (pin cushion and barrel distortion), chromatic aberration, and/or aberration of higher degree (terms above the third degree of Snell's law). In addition, the data structure 116 to 120 may be a structural aberration in the lens. Structural aberrations of the lens may include, for instance, shape aberrations (bulges or pits), lines, waste and bubbles. Each of these aberrations may affect the formed image in their own identifiable way.

After the camera unit 100 has identified an aberration characteristic of a specific optical component 110 to 114, the optical component 110 to 114 may be identified, the identification data may be stored and/or the data may be utilized for controlling the optical radiation source 300, 304, directing the optical radiation at the measurement object and processing the image.

The memory circuit may also be an RFID (Radio Frequency Identification), which may also be called an RF tag. A passive RFID does not have its own power source but it operates with energy from the reader, in this case the camera unit 100. Energy may be supplied to the RFID via a conductor from for example a battery or, in a wireless solution, the energy of the identification data inquiry signal may be utilized.

The camera unit 100 may compare the image formed by a certain optical component 110 to 114 with a reference image, which may be stored in the memory of the camera unit 100. The comparison could be made about, for example, aberration, contrast or brightness in different parts of the image. Thus, information on the optical properties, such as refractive indices of lenses, of each optical component 110 to 114 may be obtained. In addition, image errors may be corrected by, for example, controlling the optical radiation sources 300, 304 and changing the optical radiation directed at the measurement object.

The data structure 116 to 120 of each optical component 110 to 114 may thus transmit information on the optical component 110 to 114 to the camera unit 100 when at least one optical component 110 to 114 is connected to the camera unit 100. By using the information associated with each connected optical component 110 to 114, the data structure 116 to 120 may thus directly or indirectly (e.g. by means of the controller 106 or the hospital's server) control the illumination of the measurement object and the formation of an image of the organ performed with the camera unit 100.

One or more optical components 110 to 114 may also comprise a detecting cell 138, to which the optical radiation may be directed either directly or by a mirror 140, for example. The mirror 140 may be semipermeable. The detecting cell 138 may also be so small that it only covers a part of the optical radiation passing through the optical component 110 to 114, whereby optical radiation also arrives at the detector 104. An optical component 110 to 114 may comprise more than one detecting cells, and they may be in a wired connection with the controller 106 when the optical component 110 to 114 is connected to the camera unit 100. Connected to the camera unit 100, the detecting cell 138 may be activated to operate with the energy from the camera unit 100 and it may be used for forming an image of the organ. The detecting cell 138 may operate at the same or a different wavelength as the detector 104. The cell 138 operating at a different wavelength may be used for forming an image in infrared light, for instance, and the detector 104 may be used for forming an image in visible light. The mirror 140 may reflect infrared radiation very well and, at the same time, allow a considerable amount of visible light to pass through it. Image data of the detecting cell 138 and that of the detector 104 may be processed and/or combined and utilized alone or together. The detecting cell 138 may be a CCD or CMOS element, for instance.

Each optical component 110 to 114 may comprise at least one sensor 134, such as an acceleration sensor, distance sensor, temperature sensor, and/or physiological sensor. A distance sensor may measure distance to the object under examination. A physiological sensor may measure blood sugar content and/or haemoglobin, for example.

When the camera unit 100 comprises a plurality of optical radiation sources, radiation can be emitted from different sources to the object under examination according to the distance between the camera unit 100 and the object under examination. Optical radiation sources may be used, for example, in such a manner that when the camera unit 100 is further than a predetermined distance away from the eye, the source of visible light illuminates the eye. On the other hand, when the camera unit is closer than a predetermined distance from the eye, the infrared source illuminates the eye. The illumination means of the eye may thus be a function of distance.

An acceleration sensor may be used, for instance, for implementing a function in which a first image is taken from the fundus of the eye at a first moment when a certain optical radiation source emits light towards the measurement object and at least one other image is taken by using a different optical radiation source at another moment when the camera unit is in the same position with respect to the eye as when the first image was taken. Since a hand-held camera unit is shaking in the hand, the position of the camera unit with respect to the eye changes all the time. By integrating the acceleration vector $\vec{a}$ of the camera unit into a velocity vector $\vec{v}$, wherein

$$\vec{v} = \int_{t_0}^{t_1} \vec{a}\,dt,$$ and by converting the velocity vector $\vec{v}$ into a spatial position vector $\vec{x}$, wherein $\vec{x}$ t, the location of the camera unit may be determined at any moment. If the location is the same when the first and the second image are taken, the first and the second image may differ from one another in that they have been taken by using different wavelengths. Another difference may be that the shadows in the images may be cast in different directions, because the different optical radiation sources may be situated in different locations in the optical component. Different wavelengths and shadows in different directions may provide information on the measurement object.

In the case of Figure 1, where no optical component 110 to 114 is attached to the camera unit 100, no image can necessarily be formed with the camera unit 100, or it can be used for forming an image of skin, for example.

Figure 2 illustrates how an image is formed of the eye. In this case, an optical component 110 suitable for forming an image of the fundus of the eye is attached to the camera unit 100. The data structure 116 of the optical component 110 suitable for forming an image of the eye may, by means of a mechanical connection with the counterpart 122, representing information associated with said optical component and characteristic of this component, switch on one or more optical radiation sources 124 at the front part of the camera unit 100 in order to illuminate the eye. Alternatively, the radiation source 124 may be switched on in such a manner that the information associated with the optical component 110 of the data structure 116 is transmitted via a conductor or wirelessly to the counterpart 122 of the camera unit 100 and from there to the controller 106 or directly to the controller 106, which sets the radiation source 124 into operation on the basis of the information associated with the optical component 110. The radiation source 124 may be switched on automatically. In this case, the optical radiation source 126 inside the camera unit 100 may be switched on or off correspondingly. The radiation sources 124, 126 may be radiators of light in the visible region or of infrared radiation, for example.

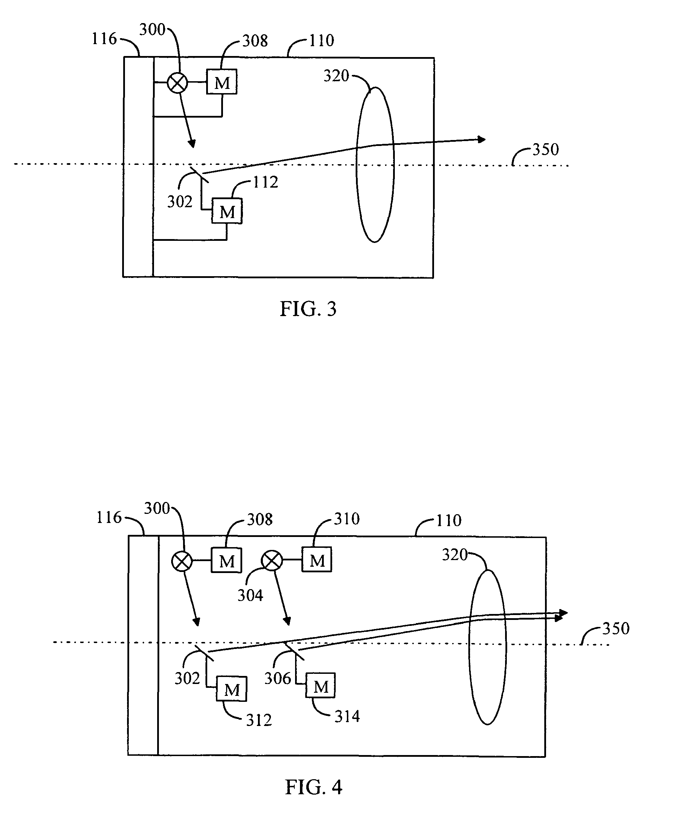

Let us now view in more detail the optical component 110 for forming an image of the eye by means of Figure 3. In Figure 3, the optical component 110 comprises one optical radiation source 300 but the optical component 110 may in general have a plurality of optical radiation sources (Figure 4). Each optical radiation source 300 may be located inside the optical component 110, and each optical radiation source 300 obtains its electric power from the camera unit 100. The optical component 110 may also comprise an optical radiation control structure 302. The optical radiation source 300 applies optical radiation to the optical radiation control structure 302, directing the radiation from the optical radiation source 300 through a lens unit 320 of the optical component 110 towards the eye under examination. The optical radiation control structure 302 may be a mirror or a prism, which is arranged non-axially to the optical axis 350 of the optical component 110 and which applies optical radiation towards the eye in a direction diverging from the optical axis 350 of the optical component 110. The optical component 300 also comprises an objective lens 320, through which the optical radiation is directed at the organ under examination.

The radiation source 300 may be switched on automatically when the optical component 110 is attached to the camera unit 100. In this case, the optical radiation source 126 inside the camera unit 100 may be switched on or off and the optical radiation source 124 may be switched off. The radiation source 300 may be a radiator of light in the visible region or of radiation in the infrared region, for example.

Figure 4 shows a solution wherein there are several optical radiation sources 300, 304 and optical radiation control structures 302, 306. All optical radiation sources 300, 304 may operate in the same wavelength region, but it is also possible that at least two optical radiation sources 300, 304 operate in different wavelength regions. Also the optical radiation bandwidth of at least two optical radiation sources 300, 304 may differ from one another.

In an embodiment, the optical radiation from all optical radiation sources 300, 304 may be non-polarized or polarized in the same way. In addition or alternatively, the optical radiation from at least two optical radiation sources 300, 304 may differ from each other in terms of polarisation. Optical radiation polarized differently may be reflected from different objects in different ways and may thus contribute to separating and detecting different objects. If the polarisation change between the transmitted and the received optical radiation is determined at the reception, a desired property of the object may be determined on the basis of this change.

In an embodiment, the optical radiation sources 300, 304 may emit pulsed radiation or continuous radiation. Pulsed radiation may be used as flashlight, for instance. The optical power sources 300, 304 may also be set to operate independently in either pulsed or continuous mode.

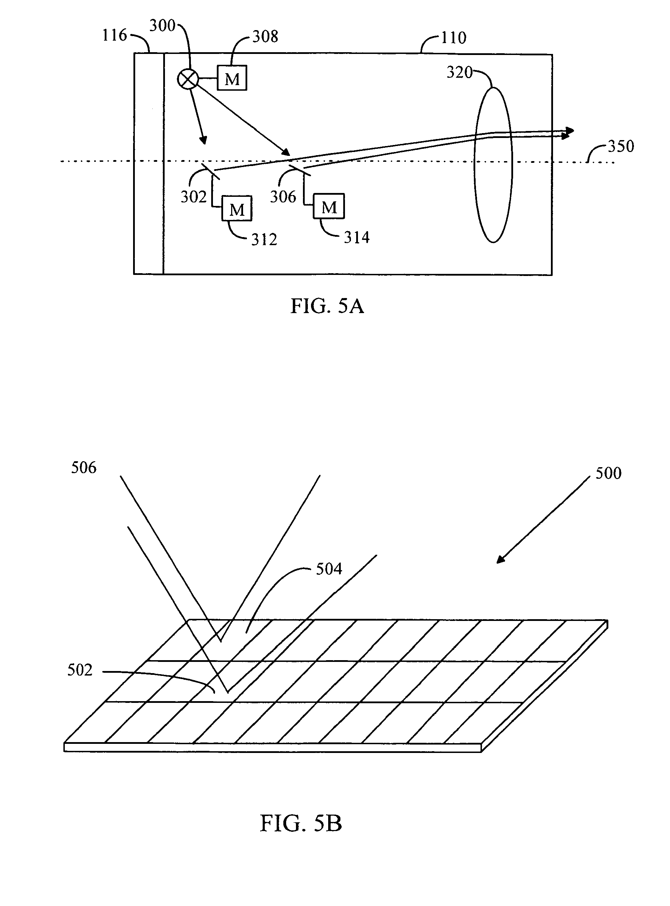

In an embodiment, each optical radiation source 300, 304 may be set to a desired position or location during image-forming. Thus, the optical radiation may be directed at the control structure 302, 306 from a desired direction. The optical radiation sources 300, 304 may be moved by means of motors 308, 310, for instance. Likewise, each optical radiation control structure 302, 306 may be set to a desired position during image-forming. The control structures 302, 306 may also be moved in their entirety by means of motors 312, 314. The control structures 302, 306 may comprise row or matrix elements, whose direction affecting the optical radiation may be controlled independently (see Figure 5B). The motors 308 to 314 may be controlled by the controller 106, which may receive the user's control commands from a user interface. Hence, the optical radiation may be directed at the eye in a desired manner from a desired direction. When an image is formed of for instance the fundus of the eye, optical radiation may be directed and/or the direction of optical radiation may be changed, whereby the fundus of the eye may be seen more clearly.

Figure 5A shows an embodiment, wherein one optical radiation source 300 emits optical radiation to two optical radiation control structures 302, 306. The optical radiation directed to both optical radiation control structures 302, 306 may have the same intensity and wavelength band, or the optical radiation source 300 may direct optical radiation with a different intensity and/or wavelength band at different control structures 302, 306. When a different type of optical radiation is directed at the optical radiation control structures 302, 306, optical radiation propagating in different directions may be filtered in a different way in the optical radiation source 300. Accordingly, both optical radiation control structures 302, 306 may direct optical radiation with the same or different intensities and wavelength bands towards the measurement object. When the same kind of optical radiation is directed at the optical radiation control structures 302, 306, optical radiation may be filtered in the optical radiation control structures 302, 306 in order to direct a different kind of optical radiation at the measurement object. The optical radiation may also be polarized in a desired manner in each control structure 302, 306, regardless of whether or not the optical radiation directed at the control structure is polarised in some way.

The control structure 302, 306 may be a digital radiation processor comprising a group of mirrors in line or matrix form, for example. The position of each mirror can be controlled. The digital radiation processor may be for example a DLP (Digital Light Processor) 500, which is shown in Figure 5B. Optical radiation 506 that arrives at different mirror elements 502, 504 may thus be reflected from each element in a desired direction.

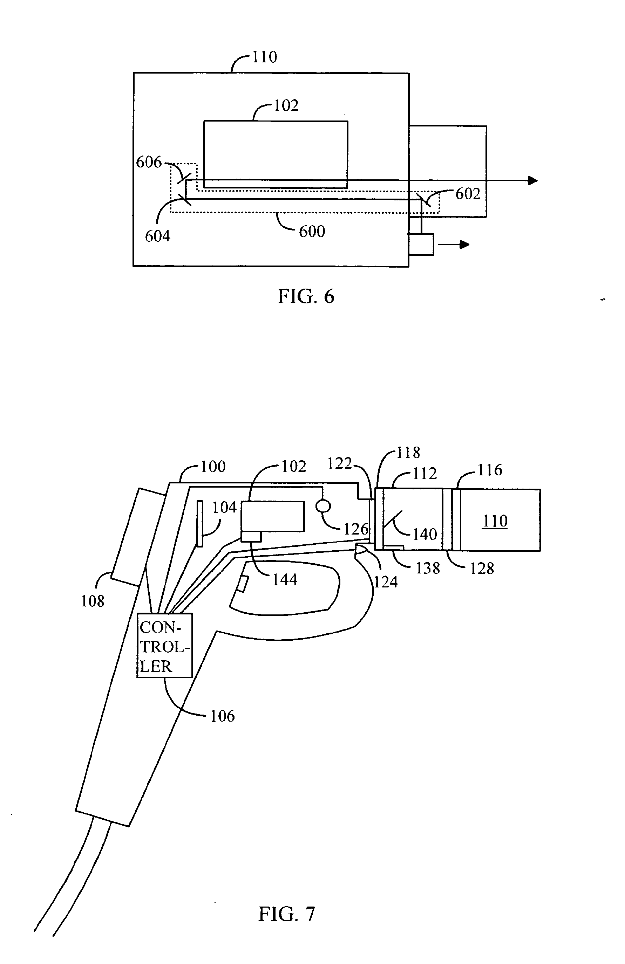

Figure 6 shows an embodiment, wherein the light source 124 in the frame of the camera unit 100 emits optical radiation both straight towards the object of which an image is formed and into the camera unit 100. The optical radiation that is directed inwards is led by means of a transfer unit 600 to be directed at the object under examination via the optics unit 102. Optical radiation may also be directed from the optics unit 102 towards the control structure 302 in the optical component 110, from which the optical radiation is directed at the organ under examination. The transfer unit 600 may comprise, for example, three reflectors 602, 604 and 606 forming a type of periscope, as shown in Figure 6. Instead of reflectors, prisms may also be used. The transfer unit 600 may also be an optical fibre or another optical radiation conductor. In the present solution, optical radiation may be directed close to the optical axis 350 of the camera unit.

Figure 7 shows an embodiment, wherein two optical components 110, 112 are attached to one another and the combination is fastened to the camera unit 100. The optical components 110, 112 are in contact with each other by means of the counterpart 128 of the optical component 112 and the data structure 116. As a consequence, when an optical component 112 suitable for forming an image of the eye is attached to an optical component 110 fitted for forming an image of the skin, an efficient device for forming an image of the fundus of the eye, for instance, can be provided. In such a case, the optical radiation from the radiation source 124 at the front part of the camera unit 100 cannot necessarily reach the eye very well. By using the information associated with the optical components 110, 112, the data structures 116, 118 of the optical components 110, 112 may then set the radiation source 124 to switch off and the radiation source 126 possibly located inside the camera unit 100 to switch on. In addition, the optical radiation source 300 may be switched on to emit optical radiation to the eye. The camera unit 100 or other data processing unit may utilize data of several data structures 116, 118 for editing image data and controlling the optical radiation sources. In addition to switching on and off, the intensity, direction or contrast of the illumination may also be adjusted, if the optical radiation of one or more radiation sources may be utilized while the eye is examined. If also the optical properties, such as focal length, polarization or optical pass band of one or more available optical components are known, the illumination properties may be affected in versatile ways.

The radiation source 300 inside the optical component 110 to 114 may be optimized to a great extent to emphasize a desired property of the object. As radiation sources both in other optical components and in the frame of the device can be directed at the measurement object at the same time, the measurement object may be illuminated with an amount of radiation required for forming an image, simultaneously emphasizing one or more desired properties.

In an embodiment, the optical component 110 to 114 directs a predetermined pattern at the measurement object. The predetermined pattern may be for instance a matrix, scale or grid. If a scale is directed at the measurement object, the sizes of the structures detected in the measurement object can be measured. For example, the size of a blood vessel, scar or tumour in the eye can be determined. The measurement can be performed by calculations on the basis of any predetermined pattern.

When a predetermined pattern is directed at the surface of the eye, it is possible to measure intraocular pressure. Intraocular pressure is usually 10 to 21 mmHg. However, the pressure will be higher if too much aqueous humour is produced in the surface cell layer of the ciliary body or humour drains too slowly through the trabecular meshwork in the anterior chamber angle into the Schlemm's canal and further to the venous circulation. During the measurement, a desired air spray may be directed at the eye from a known distance and with a predetermined or pre-measured pressure. The lower the pressure in the eye is, the more the air spray distorts the surface of the eye. The distortion produced by the air spray also causes that the predetermined pattern reflected from the surface of the eye changes. The shape of the pattern may be detected by the detector 104, and the pattern may be processed and measured by the controller 106 or an external computer. Since the force applied by the pressure to the surface of the eye may be determined on the basis of the measured or known variables, the measured change of the predetermined pattern may be used for determining the pressure that the eye must have had to enable the measured change.

In an embodiment, the formed image is coloured in a desired manner completely or partly. The colouring, as well as at least some of other procedures associated with the forming of an image, may be performed in the camera unit 100, in a separate computer 810, a docking station, a hospital's base station or a hospital's server. The colouring may be for example such that when an image is formed of the eye, the object is illuminated with orange light, when an image is formed of the ear, it is illuminated with red light, and when an image is formed of the skin, with blue light. In image processing, the image may also be edited into an orange, red or blue image.

In an embodiment, the information associated with the optical component 110 to 114 may be used for determining the information on the object of which an image is formed, such as the eye, nose, mouth, ear, skin, etc., since each optical component 110 to 114 alone or together with one or more predetermined optical component 110 to 114 may be intended for forming an image of a predetermined organ. Thus, for example, the optical component for examining the eye allows the information "eye optics" or "image of an eye" to be automatically attached to the image. As the camera unit 100 identifies the object of which an image is formed on the basis of information associated with one or more optical components 110 to 114, the camera unit 100 may by image processing operations automatically identify predetermined patterns in the object of which an image is formed and possibly mark them with colours, for instance. When the image is displayed, the marked-up sections, which may be for instance features of an illness, can be clearly distinguished.

Information received from one or more optical components may be used for monitoring how the diagnosis succeeds. If the patient has symptoms in the eye, but the camera unit 100 was used for forming an image of the ear, it can be deduced that this was not the right way of acting. It is also possible that the hospital's server has transmitted information on the patient and his/her ailment in DICOM (Digital Imaging and Communications in Medicine) format, for instance, to the camera unit 100. Thus, the camera unit 100 only forms an image of the organ about which the patient has complained, in this case the eye. If some other optical component 110 to 114 than the optical component suitable for forming an image of the object (eye) that is ailing is attached to the camera unit 100, the camera unit 100 warns the cameraman with a sound signal and/or a warning signal on the display of the camera unit 100.

For example, a hospital's patient data system collecting images by using the information received from one or more optical components can produce both statistics and billing data.

In an embodiment, by using the information associated with one or more optical components 110 to 114 attached to the camera unit 100, the illumination of the environment may be controlled and the illumination of the organ of which an image is formed may thus also be affected. Thus, the information on the optical component 110 to 114 is transferred, for instance, to the controller controlling the illumination of the examination room. The illumination of the examination room may also be controlled in other ways such that, for example, the illumination may be increased or reduced, or the colour or shade of the colour of the illumination may be controlled. When the camera unit 100 and the object of which an image is formed are close to the light source of the examination room, the light source may be dimmed, for example. Accordingly, if the camera unit 100 is far away from the light source, the light source may be adjusted to illuminate more intensely.

In an embodiment, the location of the optical component 110 to 114 fastened to the camera unit 100 may be determined by using, for example, one or more UWB (Ultra Wide Band) or WLAN transmitters (Wireless Local Area Network). Each transmitter transmits an identification, and the location of each transmitter is known. By using one transmitter, the position of the optical component 110 to 114 may be determined on the basis of the coverage of the transmitter, and on the basis of transmissions of two transmitters, the location of the optical component 110 to 114 may often be determined more precisely, but resulting in two alternative locations, and on the basis of transmissions of three or more transmitters, the location of the optical component 110 to 114 may be determined by triangulation quite accurately and more precisely than the coverage of the transmission. After the location of the used optical component 110 to 114 is determined, the illumination of the examination room may be controlled so that the information on the location of the optical component 110 to 114 is transferred, for instance, to the controller controlling the illumination of the examination room. The illumination of the examination room may be controlled in the same way as in the previous example. For instance, if the optical component 110 to 114 is used in a place that is known to be located next to the patient table, the illumination of the patient table and its surroundings may be increased (or reduced) automatically. Moreover, the image taken by the camera unit 100 may be attached with the information that the image is taken next to the patient table.

In an embodiment, the optical component 110 to 114 comprises an accelerator sensor, which may determine the position of the camera unit 100. After the position of the camera unit 100 is determined, the position information may be used for controlling the illumination of the patient room such that the information on the position of the camera unit 100 is transferred, for instance, to the controller controlling the illumination of the examination room, like in the previous examples. Acceleration sensors may be used for determining accelerations of the camera unit 100, and by integrating the accelerations, the velocity of the camera unit may be determined, and by integrating the velocity, the location of the camera unit may be determined if the camera unit has been located in a predetermined place at the starting moment. Consequently, the location of the camera unit can be determined by this measurement alone or together with the previous measurements three-dimensionally. The lights of the examination room may thus be controlled three-dimensionally to be suitable for taking images.

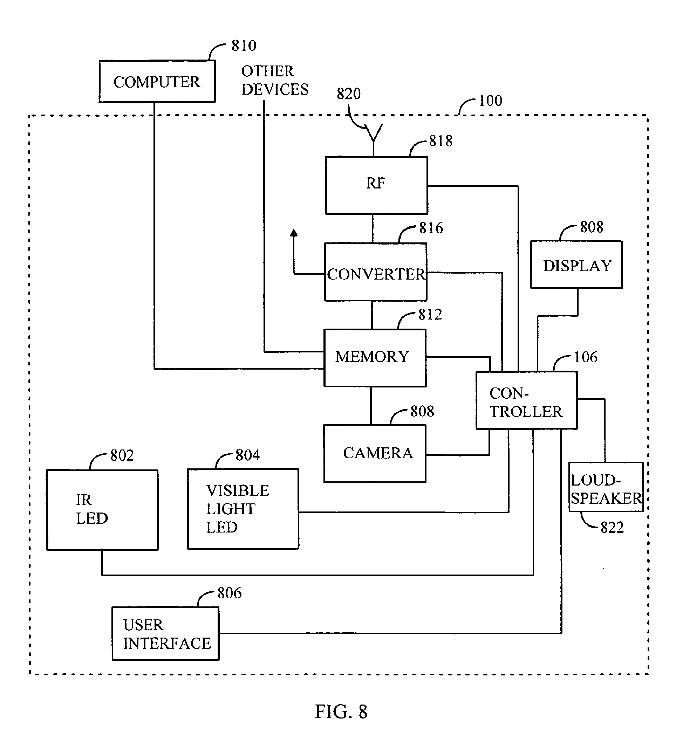

Let us view a block diagram of the examination device by means of Figure 8. The examination device may comprise an infrared radiation source 802, a visible light source 804, a user interface 806, a camera part 808, a controller 106 and a memory 812. The camera part 808 comprises, for instance, a detector 104. The controller 106, which may comprise a processor and memory, may control the operation of the camera part 808. The controller 106 may receive the information associated with one or more optical components 110 to 114 and control the image formation by adjusting illumination, image brightness, contrast, colour saturation, colour, etc. The image may be transferred from the camera part 808 to the memory 812, from which the image may be transferred onto the display of the user interface 806, to a loudspeaker 822 and/or elsewhere, controlled by the controller 106. Still pictures or video image taken from the object of which an image is formed may be stored in the memory 812, which may be a flash type of memory or a repeatedly detachable and attachable memory, such as an SD (Secure Digital) memory card. The memory 812 may also be located in the optical component 110 to 114. The converter 816 may convert the format of the signal from the memory 812. The converter 816 may also convert the format of the signal from radio-frequency parts 818. The examination device may transmit and receive radio-frequency signals with an antenna 820. The radio-frequency parts 818 may mix the baseband signal to be transmitted to the radio frequency, and the radio-frequency parts 818 may mix the received radio-frequency signal down to the baseband.

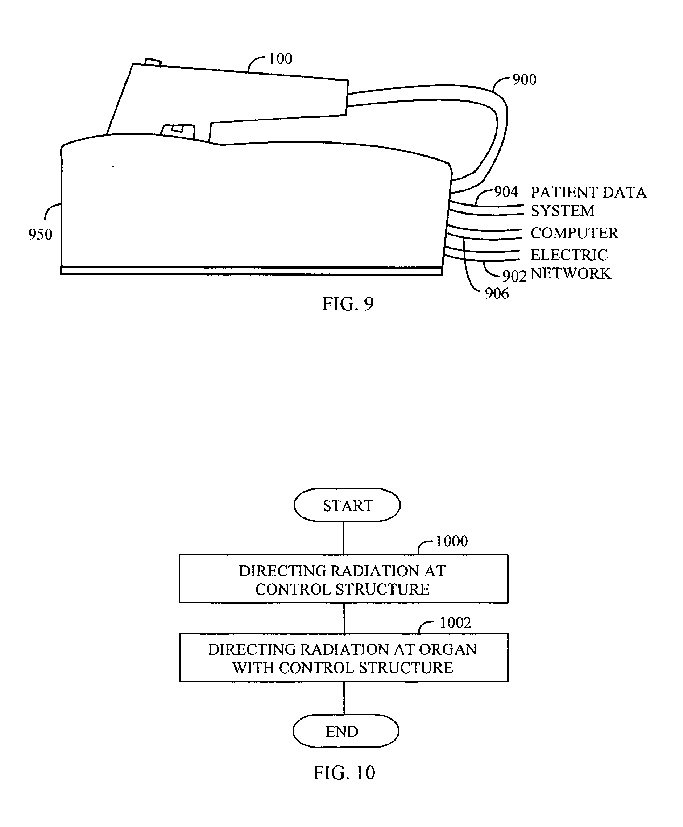

Figure 9 shows the camera unit 100 connected to a docking station 950. The examination device may comprise just the camera unit 100 or both the camera unit 100 and the docking station 950. The docking station 950 may be connected by a conductor 902 to a general electrical network, from which the docking station 950 takes electric power and uses it for its own operation or may convert it into a format required by the camera unit 100. Between the camera unit 100 and the docking station 950 there is a cable 900, along which the docking station 950 supplies the camera unit 100 with the electric power required to charge the battery of the camera unit 100, for instance. The docking station 950 may also be shaped in such a manner that the camera unit 100 may be set firmly in its place in the docking station 950 when the camera unit 100 is not being used for examining the organ. Also the docking station 950 may comprise a battery. The docking station 950 may be connected by a conductor 904 to a data network of a patient data system, connected to the network, or the docking station 950 may be in wireless connection with the hospital's base station acting as an access point in data transfer with the hospital's server. In addition, the docking station 950 may communicate with a PC, for example, by a cable 906.

Figure 10 shows a flow diagram of the method. In step 1000, optical radiation is directed by at least one optical radiation source to at least one optical radiation control structure, which is located non-axially to the optical axis of the optical component. In step 1002, optical radiation is directed by each optical radiation control structure at the organ under examination in a direction diverging from the optical axis of the optical component.

Although the invention is described above with reference to the example according to the accompanying drawings, it is obvious that the invention is not restricted thereto but may be varied in many ways within the scope of the attached claims.

**Claims**

1. A method for illuminating an organ, wherein a camera unit (100) is used for forming an electric image of the organ, the method employing at least one optical component (110 to 114), which is connectable to the camera unit (100) and comprises at least one optical radiation source (300, 304) and at least one optical radiation control structure (302, 306), each optical component (110 to 114) including a data structure (116 to 120) containing information associated with the optical component (110 to 114);
directing (1000) optical radiation with the at least one optical radiation source (300, 304) to the at least one optical radiation control structure (302, 306), which is located non-axially to the optical axis (350) of the optical component (110 to 114);
directing (1002) optical radiation with each optical radiation control structure (302, 306) at the organ under examination in a direction diverging from the optical axis (350) of the optical component (110 to 114);
transferring information associated with the optical component (110 to 114) from the data structure (116 to 120) to the camera unit (100) when at least one optical component (110 to 114) is connected to the camera unit (100); and
controlling the formation of an image of the organ by the camera unit (100) on the basis of the information associated with one or more optical components (110 to 114).

2. A method as claimed in claim 1, **characterized by** directing optical radiation at the eye, which is the organ under examination.

3. A method as claimed in claim 1, **characterized by** setting the optical radiation source (300, 304) to illuminate the organ under examination on the basis of the information associated with the one or more optical components.

4. A method as claimed in claim 3, **characterized by** controlling the intensity of each optical radiation source (300, 302).

5. A method as claimed in claim 3, **characterized by** controlling the optical bandwidth of each optical radiation source (300, 302).

6. A method as claimed in claim 1, **characterized by** setting the optical radiation source (300, 304) to emphasize a desired feature of the organ under examination by means of its optical radiation.

7. A device for forming an image of an organ, the device comprising a camera unit (100) for forming an electric image of the organ, wherein:

the device comprises a group of optical components (110 to 114), the group comprising at least one optical component (110 to 114), each optical component (110 to 114) being connectable to the camera unit (100);
each optical component (110 to 114) comprises at least one optical radiation source (300, 304), at least one optical radiation control structure (302, 306) and a data structure (116 to 120) containing information associated with the optical component (110 to 114);
the device is arranged to transfer information associated with the optical component (110 to 114) from the data structure (116 to 120) to the camera unit (100) when at least one optical component (110 to 114) is connected to the camera unit (100);
the at least one optical radiation source (300, 304) is arranged to direct optical radiation at the at least one optical radiation control structure (302, 306) located non-axially to the optical axis (350) of the optical component (110 to 114);
each optical radiation control structure (302, 306) is arranged to direct optical radiation from the optical radiation source (300, 304) at the organ in a direction diverging from the optical axis (350) of the optical component (110 to 114); and
the device is arranged to control the formation of an image of the organ by the camera unit (100) on the basis of the information associated with one or more optical components (110 to 114).

8. A device as claimed in claim 7, **characterized in that** the device is an opthalmoscope for illuminating the eye and forming an image thereof.

9. A device as claimed in claim 7, **characterized in that** the camera unit (100) comprises a detector (104) and an optics unit (102) arranged to form, together with the at least one optical component (110 to 114), an image to the detector (104) of the camera unit (100).

10. A device as claimed in claim 9, **characterized in that** the device is arranged to set the optical radiation source (300, 304) to illuminate the organ under examination on the basis of the information associated with the one or more optical components.

11. A device as claimed in claim 7, **characterized in that** the device is arranged to control the intensity of each optical radiation source (300, 302).

12. A device as claimed in claim 7, **characterized in that** the device is arranged to control the optical bandwidth of each optical radiation source (300, 302).

13. A device as claimed in claim 9, **characterized in that** the device is arranged to set the optical radiation source (300, 304) to emphasize a desired feature of the organ under examination by means of its optical radiation.


**Patentansprüche**

1. Ein Verfahren zur Beleuchtung eines Organs, wobei eine Kameraeinheit (100) verwendet wird, um ein elektrisches Bild des Organs zu bilden, wobei das Verfahren mindestens eine optische Komponente (110 bis 114) einsetzt, welche an die Kameraeinheit (100) anschließbar ist und mindestens eine optische Strahlungsquelle (300, 304) und mindestens eine Steuerstruktur der optischen Strahlung (302, 306) aufweist, wobei jede optische Komponente (110 bis 114) eine Datenstruktur (116 bis 120) aufweist, welche mit der optischen Komponente (110 bis 114) assoziierte Information enthält;
optische Strahlung mit der mindestens einen optischen Strahlungsquelle (300, 304) auf die mindestens eine Steuerstruktur der optischen Strahlung (302, 304) gerichtet wird (1000), welche sich nicht-axial zu der optischen Achse (350) der optischen Komponente (110 bis 114) befindet;
optische Strahlung mit jeder Steuerstruktur der optischen Strahlung (302, 304) auf das zu untersuchende Organ in einer, von der optischen Achse (350) der optischen Komponente (110 bis 114) abweichenden Richtung gerichtet wird (1002);
mit der optischen Komponente (110 bis 114) assoziierte Information von der Datenstruktur (116 bis 120) an die Kameraeinheit (100) übertragen wird, wenn mindestens eine optische Komponente (110 bis 114) an die Kameraeinheit (100) angeschlossen ist; und
die Bildung des Bildes des Organs durch die Kameraeinheit (100) auf der Basis der mit einer oder mehreren optischen Komponenten (110 bis 114) assoziierten Information gesteuert wird.

2. Ein Verfahren gemäß dem Patentanspruch 1, **dadurch gekennzeichnet, dass** die optische Strahlung auf das Auge gerichtet wird, welches das zu untersuchende Organ ist.

3. Ein Verfahren gemäß dem Patentanspruch 1, **dadurch gekennzeichnet, dass** die optische Strahlungsquelle (300, 304) eingerichtet ist, das zu untersuchende Organ auf der Basis der mit einer oder mehreren optischen Komponenten assoziierten Information zu beleuchten.

4. Ein Verfahren gemäß dem Patentanspruch 3, **dadurch gekennzeichnet, dass** die Intensität jeder optischen Strahlungsquelle (300, 302) gesteuert wird.

5. Ein Verfahren gemäß dem Patentanspruch 3, **dadurch gekennzeichnet, dass** die optische Bandbreite jeder optischen Strahlungsquelle (300, 302) gesteuert wird.

6. Ein Verfahren gemäß dem Patentanspruch 1, **dadurch gekennzeichnet, dass** die optische Strahlungsquelle (300, 304) eingerichtet ist, eine gewünschte Eigenschaft des zu untersuchenden Organs mit Hilfe ihrer optischen Strahlung hervorzuheben.

7. Eine Vorrichtung zur Bildung eines Bildes eines Organs, wobei die Vorrichtung eine Kameraeinheit (100) zur Bildung eines elektrischen Bildes des Organs aufweist, wobei:

die Vorrichtung eine Gruppe von optischen Komponenten (110 bis 114) aufweist, wobei die Gruppe mindestens eine optische Komponente (110 bis 114) aufweist, wobei jede optische Komponente (110 bis 114) an die Kameraeinheit (100) anschließbar ist;
jede optische Komponente (110 bis 114) mindestens eine optische Strahlungsquelle (300, 304), mindestens eine Steuerstruktur der optischen Strahlung (302, 306) und eine Datenstruktur (116 bis 120) aufweist, welche mit der optischen Komponente (110 bis 114) assoziierte Information enthält;
die Vorrichtung angeordnet ist, mit der optischen Komponente (110 bis 114) assoziierte Information von der Datenstruktur (116 bis 120) an die Kameraeinheit (100) zu übertragen, wenn mindestens eine optische Komponente (110 bis 114) an die Kameraeinheit (100) angeschlossen ist;
die mindestens eine optische Strahlungsquelle (300, 304) angeordnet ist, optische Strahlung auf die mindestens eine Steuerstruktur der optischen Strahlung (302, 304) zu richten, welche sich nicht-axial zu der optischen Achse (350) der optischen Komponente (110 bis 114) befindet;
jede Steuereinheit der optischen Strahlung (302, 304) angeordnet ist, optische Strahlung von der Strahlungsquelle der optischen Strahlung (300, 304) auf das Organ in einer, von der optischen Achse (350) der optischen Komponente (110 bis 114) abweichenden Richtung zu richten; und

die Vorrichtung angeordnet ist, die Bildung eines Bildes des Organs durch die Kameraeinheit (100) auf der Basis der mit einer oder mehreren optischen Komponenten (110 bis 114) assoziierten Information zu steuern.

8. Eine Vorrichtung gemäß dem Patentanspruch 7, **dadurch gekennzeichnet, dass** die Vorrichtung ein Ophthalmoskop zur Beleuchtung des Auges und zur Bildung eines Bildes davon ist.

9. Eine Vorrichtung gemäß dem Patentanspruch 7, **dadurch gekennzeichnet, dass** die Kameraeinheit (100) einen Detektor (104) und eine Optikeinheit (102) aufweist, die angeordnet sind, zusammen mit der mindestens einen optischen Komponente (110 bis 114) ein Bild an den Detektor (104) der Kameraeinheit (100) zu bilden.

10. Eine Vorrichtung gemäß dem Patentanspruch 9, **dadurch gekennzeichnet, dass** die Vorrichtung angeordnet ist, die optische Strahlungsquelle (300, 304) einzurichten, das zu untersuchende Organ auf der Basis der mit der einen oder den mehreren optischen Komponenten assoziierten Information zu beleuchten.

11. Eine Vorrichtung gemäß dem Patentanspruch 7, **dadurch gekennzeichnet, dass** die Vorrichtung angeordnet ist, die Intensität jeder optischen Strahlungsquelle (300, 302) zu steuern.

12. Eine Vorrichtung gemäß dem Patentanspruch 7, **dadurch gekennzeichnet, dass** die Vorrichtung angeordnet ist, die optische Bandbreite jeder optischen Strahlungsquelle (300, 302) zu steuern.

13. Eine Vorrichtung gemäß dem Patentanspruch 9, **dadurch gekennzeichnet, dass** die Vorrichtung angeordnet ist, die optische Strahlungsquelle (300, 304) einzurichten, eine gewünschte Eigenschaft des zu untersuchenden Organs mit Hilfe ihrer optischen Strahlung hervorzuheben.

## Revendications

1. Procédé d'éclairage d'un organe, dans lequel une unité de caméra (100) est utilisée pour former une image électrique de l'organe, le procédé comportant l'emploi d'au moins un composant optique (110 à 114), lequel peut être connecté à l'unité de caméra (100), et comprend au moins une source de rayonnement optique (300, 304) et au moins une structure de commande de rayonnement optique (302, 306), chaque composant optique (110 à 114) incluant une structure de données (116 à 120) contenant des informations associées au composant optique (110 à 114) ;

l'orientation (1000) du rayonnement optique avec la au moins une source de rayonnement optique (300, 304), vers la au moins une structure de commande de rayonnement optique (302, 306), laquelle est située de manière non axiale à l'axe optique (350) du composant optique (110 à 114) ;

l'orientation (1002) du rayonnement optique avec chaque structure de commande de rayonnement optique (302, 306), au niveau de l'organe examiné, dans une direction qui s'écarte de l'axe optique (350) du composant optique (110 à 114) ;

le transfert d'informations associées au composant optique (110 à 114), de la structure de données (116 à 120) à l'unité de caméra (100), lorsqu'au moins un composant optique (110 à 114) est connecté à l'unité de caméra (100) ; et

la commande de la formation d'une image de l'organe par l'unité de caméra (100) sur la base des informations associées à un ou plusieurs composants optiques (110 à 114).

2. Procédé selon la revendication 1, **caractérisé par** l'orientation du rayonnement optique au niveau de l'oeil, lequel est l'organe examiné.

3. Procédé selon la revendication 1, **caractérisé par** la configuration de la source de rayonnement optique (300, 304) afin d'éclairer l'organe examiné sur la base des informations associées audit un ou auxdits plusieurs composants optiques.

4. Procédé selon la revendication 3, **caractérisé par** la commande de l'intensité de chaque source de rayonnement optique (300, 302).

5. Procédé selon la revendication 3, **caractérisé par** la commande de la bande passante optique de chaque source de rayonnement optique (300, 302).

6. Procédé selon la revendication 1, **caractérisé par** la configuration de la source de rayonnement optique (300, 304) afin de souligner une caractéristique désirée de l'organe examiné, au moyen de son rayonnement optique.

7. Dispositif destiné à former une image d'un organe, le dispositif comprenant une unité de caméra (100) pour former une image électrique de l'organe, dans lequel :

le dispositif comprend un groupe de composants optiques (110 à 114), le groupe comprenant au moins un composant optique (110 à 114), chaque composant optique (110 à 114) pouvant être connecté à l'unité de caméra (100) ;

chaque composant optique (110 à 114) comprend au moins une source de rayonnement optique (300, 304), au moins une structure de commande de rayonnement optique (302, 306) et une structure de données (116 à 120) contenant des informations associées au composant optique (110 à 114) ;

le dispositif est agencé de façon à transférer des informations associées au composant optique (110 à 114), de la structure de données (116 à 120) à l'unité de caméra (100), lorsqu'au moins un composant optique (110 à 114) est connecté à l'unité de caméra (100) ;

la au moins une source de rayonnement optique (300, 304) est agencée de façon à orienter le rayonnement optique au niveau de la au moins une structure de commande de rayonnement optique (302, 306) située de manière non axiale à l'axe optique (350) du composant optique (110 à 114) ;

chaque structure de commande de rayonnement optique (302, 306) est agencée de façon à orienter le rayonnement optique provenant de la source de rayonnement optique (300, 304) au niveau de l'organe, dans une direction qui s'écarte de l'axe optique (350) du composant optique (110 à 114) ; et

le dispositif est agencé de façon à commander la formation d'une image de l'organe par l'unité de caméra (100) sur la base des informations associées à un ou plusieurs composants optiques (110 à 114).

8. Dispositif selon la revendication 7, **caractérisé en ce que** le dispositif est un ophtalmoscope destiné à éclairer l'oeil et à former une image de celui-ci.

9. Dispositif selon la revendication 7, **caractérisé en ce que** l'unité de caméra (100) comprend un détecteur (104), et une unité optique (102) agencée de façon à former, conjointement avec le au moins un composant optique (110 à 114), une image sur le détecteur (104) de l'unité de caméra (100).

10. Dispositif selon la revendication 9, **caractérisé en ce que** le dispositif est agencé de façon à configurer la source de rayonnement optique (300, 304) afin d'éclairer l'organe examiné sur la base des informations associées audit un ou auxdits plusieurs composants optiques.

11. Dispositif selon la revendication 7, **caractérisé en ce que** le dispositif est agencé de façon à commander l'intensité de chaque source de rayonnement optique (300, 302).

12. Dispositif selon la revendication 7, **caractérisé en ce que** le dispositif est agencé de façon à commander la bande passante optique de chaque source de rayonnement optique (300, 302).

13. Dispositif selon la revendication 9, **caractérisé en ce que** le dispositif est agencé de façon à configurer la source de rayonnement optique (300, 304) afin de souligner une caractéristique désirée de l'organe examiné au moyen de son rayonnement optique.

FIG. 1

FIG. 2

FIG. 3

FIG. 4

FIG. 5A

FIG. 5B

FIG. 6

FIG. 7

FIG. 8

FIG. 9

FIG. 10

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20020003608 A
- US 5155509 A
- US 4932774 A
- WO 2007076479 A
- FI 107120
- FI 200212233
- FI 20075499